# DEMANDE DE BREVET EUROPEEN

(11) **EP 3 763 437 A1**
(43) Date de publication de la demande: **13.01.2021**
(21) Numéro de dépôt: 19305926.8
(22) Date de dépôt: 09.07.2019
(51) Int. Cl.: B01J 19/02, C07C 7/08, C07C 7/12, C07C 11/167, C10G 75/00, C23C 22/62, C23G 1/08, C10G 9/16

(54) **PROCEDE DE PASSIVATION DES SURFACES METALLIQUES D'UNE UNITE DE SEPARATION DU 1,3-BUTADIENE**

(71) Demandeur: Total Raffinage Chimie, 92400 Courbevoie (FR)
(72) Inventeur: GALLIOT, Ludovic, 76600 LE HAVRE (FR); THORET-BAUCHET, Jean-Pierre, 1180 UCCLE BRUXELLES (BE); RENAUD, Lionel, 76930 OCTEVILLE SUR MER (FR)
(74) Mandataire: Blaise, Lucie

(57) **Abrégé**

L'invention concerne un procédé de passivation des surfaces métalliques d'une unité de séparation du 1,3-butadiène. Le procédé comprend une étape de décapage en présence d'une solution acide de pH de 3 à 5 constituée d'une solution aqueuse d'acide citrique et d'ammoniaque, à une température de 50°C à 70°C, pendant une durée suffisante pour éliminer les oxydes de fer présents. Le procédé comprend également une étape de passivation en présence d'une solution alcaline constituée d'une solution aqueuse de nitrite de sodium contenant du dioxygène dissous, pendant 12 à 48 heures à une température supérieure à 70°C, la solution aqueuse de nitrite de sodium contenant de 0,3 à 10% en masse de nitrite de sodium et de 200 ppb en masse de dioxygène à la limite de solubilité du dioxygène. L'invention concerne également un procédé de séparation du 1,3-butadiène dans lequel l'unité de séparation est traitée par ce procédé de passivation.

## Description

### Domaine de l'invention

L'invention concerne un procédé de passivation des surfaces métalliques d'une unité de séparation du 1,3-butadiène. L'invention concerne également un procédé de séparation du 1,3-butadiène.

### Art antérieur

Dans le domaine du raffinage du pétrole, les unités butadiènes traitent la fraction C4 (hydrocarbures à 4 atomes de carbone) issue d'un vapocraqueur. La coupe C4 est constituée en général à peu près pour moitié de 1,3-butadiène et contient également en proportions notables de l'isobutène et du 1-butène. Une unité butadiène comprend usuellement une section de séparation du butadiène, généralement par distillation.

La formation de rouille à l'intérieur de la section de séparation du butadiène initie des réactions de polymérisation parasites en présence de butadiène concentré conduisant à la formation de polymère dit « pop-corn ». La formation de ce type de polymère est très rapide, ce qui peut poser des problèmes de sécurité et nécessiter l'arrêt de l'unité. En outre, une fois formé, ce polymère est difficile à éliminer. Les mêmes phénomènes se produisent également dans les trains de séparation à froid des vapocraqueurs. Ces trains de séparation comprennent plusieurs sections de séparation par distillation ayant pour objectif d'isoler les différentes coupes ainsi que les différents produits fabriqués dans le vapocraqueur. Dans certaines de ces sections de séparation, la coupe C4 est relativement concentrée, tel que par exemple en fond dépropaniseur et dans l'intégralité du débutaniseur, et peut conduire à la formation de pop-corn en présence de rouille. En fonctionnement, la présence de dioxygène est à proscrire, le dioxygène pouvant conduire à la formation de peroxydes qui peuvent catalyser la réaction de polymérisation de ces polymères « pop-corn ».

Pour limiter la formation de rouille, des traitements de passivation sont réalisés afin de rendre les surfaces métalliques internes moins réactives. L'objectif est de rendre la surface la plus inerte possible, de manière durable dans le temps, la formation de « pop-corn » et autres polymères plus ou moins rapidement étant un indicateur a posteriori de la qualité du traitement réalisé. Ces traitements de passivation sont le plus souvent réalisés sous atmosphère de diazote. Ceci peut permettre d'éviter la création de microbulles de dioxygène dans la couche de passivation susceptibles de favoriser la formation de ces polymères pop-corn. Cela peut également permettre la formation d'une couche de passivation dans les conditions redox du procédé (autrement dit sans O₂). En effet, il existe un risque qu'une couche créée dans des conditions oxydantes (en présence de O₂) se transforme (soit réduite) dans les conditions réductrices du procédé et ne protège plus les parois.

Les traitements de passivation de surface peuvent être partiellement efficaces, du fait des difficultés de circulation du fluide et du maintien en température pendant toute la durée du processus de traitement.

On observe en outre encore la formation de polymère dit « pop-corn ». Il existe donc un besoin pour un procédé de passivation amélioré d'une unité de séparation du butadiène.

### Résumé de l'invention

A cet effet, un premier objet de l'invention concerne un procédé de passivation des surfaces métalliques d'une unité de traitement d'une coupe contenant du 1,3-butadiène, comprenant les étapes suivantes :
(i) une étape de décapage dans laquelle lesdites surfaces métalliques sont mises en contact avec une solution acide présentant un pH de 3 à 5 constituée d'une solution aqueuse d'acide citrique et d'ammoniaque, et optionnellement d'un ou plusieurs inhibiteurs de corrosion, à une température comprise dans la plage allant de 50°C à 70°C pendant une durée suffisante pour éliminer les oxydes de fer présents sur lesdites surfaces métalliques,
(ii) une étape d'évacuation de la solution acide ou de neutralisation de la solution acide par une base, de préférence de l'ammoniaque,
(iii) une étape de passivation dans laquelle lesdites surfaces métalliques préalablement traitées par la solution acide sont mises en contact avec une solution alcaline constituée d'une solution aqueuse de nitrite de sodium contenant du dioxygène dissous, pendant 12 à 48 heures à une température supérieure à 70°C, la solution aqueuse de nitrite de sodium contenant de 0,3 à 10% en masse de nitrite de sodium et de 200 ppb en masse de dioxygène à la limite de solubilité du dioxygène dans la solution alcaline.

Une unité de traitement d'une coupe contenant du 1,3-butadiène est par exemple une unité de séparation. De telles unités de séparation sont présentes dans les unités butadiènes traitant les fractions C4 issues de vapocraquage et dans les unités de vapocraquage produisant ces fractions C4 ou les fractions C3, en particulier au niveau des trains de séparation de celles-ci.

Les surfaces métalliques d'une unité de traitement du 1,3-butadiène sont en général des surfaces en acier, par exemple en acier non allié tel que défini dans la norme NF EN 10027-1 : 2017.

L'étape (i) de décapage du procédé selon l'invention permet de solubiliser les oxydes de fer, l'acide citrique et l'ammoniaque présents assurant une chélation du fer dissous évitant ainsi qu'il ne précipite à l'intérieur de l'unité de séparation et qu'il ne l'encrasse.

L'étape (iii) de traitement alcalin assure une passivation des surfaces métalliques débarrassées des oxydes de fer. Cette étape permet de former une couche d'oxyde de fer stable et inerte protégeant la surface de la corrosion. Malgré la présence de dioxygène dissous lors de cette étape, on n'observe pas d'augmentation de formation de pop-corn lors de l'utilisation de l'unité de séparation.

Dans un mode de réalisation, les surfaces métalliques peuvent être soumises à une étape de dégraissage préalablement à l'étape (i) de décapage.

Un deuxième objet de l'invention concerne un procédé de traitement d'une coupe contenant du 1,3-butadiène dans une unité de traitement, comprenant :
(a) une étape de passivation des surfaces métalliques de l'unité de traitement par mise en oeuvre du procédé de passivation selon l'invention,
(b) suivie d'une étape de traitement d'une coupe contenant du 1,3-butadiène par distillation.

### Description détaillée de l'invention

Le procédé de passivation selon l'invention comprend trois étapes (i) à (iii). Dans un mode de réalisation, il est constitué uniquement de ces trois étapes (i), (ii), (iii), éventuellement précédées d'une étape de dégraissage.

Notamment, la solution acide de l'étape (i) peut être évacuée au cours de l'étape (ii) avant mise en contact de la solution alcaline de l'étape (iii) avec les surfaces métalliques. Ou alors, la solution de l'étape (i) peut être conservée, l'étape (ii) est alors une étape de neutralisation du pH de la solution de l'étape (i) par ajout d'une base, de préférence par ajout d'ammoniaque, permettant par exemple d'atteindre un pH de 7.

### Etape de dégraissage

Cette étape optionnelle, à réaliser avant l'étape de décapage, peut comprendre la mise en contact des surfaces métalliques avec une solution de dégraissage.

La solution de dégraissage peut être une solution alcaline, par exemple une solution aqueuse de NaOH contenant de 0,3 à 5%, de 0,3 à 4, de 0,3 à 3, de 0,3 à 2 ou de 0,3 à 1% en masse de NaOH ou être comprise dans toute plage définie par une combinaison de ces bornes.

La solution alcaline peut en outre comprendre un ou plusieurs surfactants, en une teneur inférieure ou égale à 0,1%m, 0,05%m, ou 0,03%m, par exemple de 0,005 à 0,015%m, ou être comprise dans toute plage définie par une combinaison de ces bornes.

Dans un mode de réalisation, la solution de dégraissage est constituée uniquement d'une solution aqueuse de NaOH, et éventuellement de surfactant(s).

La mise en contact peut être réalisée à une température de 45 à 85°C, de 50 à 80°C, de 55 à 75°C ou de 60 à 70°C ou être comprise dans toute plage définie par une combinaison de ces bornes.

La mise en contact peut durer de 5 à 12 heures, par exemple de 6 à 10 heures.

Cette étape peut être réalisée sous air.

### Etape de décapage

La solution acide utilisée dans cette étape (i) est constituée d'une solution aqueuse d'acide citrique et d'ammoniaque. Optionnellement un ou plusieurs inhibiteurs de corrosion peuvent être ajoutés mais aucun autre acide ou aucun autre composé azoté n'est nécessaire.

L'acide citrique présente l'avantage d'être facilement disponible et peu coûteux. En outre, il forme des complexes avec le fer (citrates de fer) qui sont stabilisés par l'ammoniaque, limitant ainsi, voire supprimant, le risque de précipitation de fer dans la solution.

Les quantités d'acide citrique et d'ammoniaque sont ajustées afin que le pH de la solution acide soit de 3 à 5. Cette gamme de pH assure une bonne dissolution des oxydes de fer tout en limitant les risques d'attaque des surfaces métalliques par l'acidité de la solution.

La solution acide est mise en contact avec les surfaces métalliques à traiter à une température de 50°C à 70°C, pendant une durée suffisante pour éliminer les oxydes de fer présents sur lesdites surfaces métalliques. Cette durée peut être déterminée en suivant la teneur en fer de la solution, la dissolution des oxydes de fer présents sur les surfaces métalliques étant complète lorsque la teneur en fer dissous n'augmente plus. Cette durée est par exemple de 6 à 12 heures.

Lorsque l'étape de décapage est terminée, à savoir lorsqu'il n'y a plus d'oxydes de fer sur les surfaces métalliques, autrement dit quand la teneur en fer de la solution acide est stable, la solution acide est évacuée et les surfaces métalliques peuvent être passivées par mise en oeuvre de l'étape de passivation.

### Etape de passivation

La solution alcaline utilisée au cours de cette étape est constituée d'une solution aqueuse de nitrite de sodium contenant du dioxygène dissous. Cette solution aqueuse contient de 0,3 à 10% en masse de nitrite de sodium, par exemple de 1 à 8%m, de 1 à 7%m, de 1 à 5%m de 2 à 4%m ou être comprise dans toute plage définie par une combinaison de ces bornes.

La solution alcaline contient en outre une quantité de dioxygène allant de 200 ppb en masse à la limite de solubilité du dioxygène dans la solution alcaline. Cette dernière varie notamment en fonction de la température de la solution alcaline. A titre d'exemple, la limite de solubilité du dioxygène à 90°C est de l'ordre de 1600ppb en masse.

Le pH de la solution alcaline peut être de 8 à 10, par exemple de 8 à 9.

Les surfaces métalliques sont mises en contact avec la solution alcaline pendant 12 à 48 heures, par exemple de 12 à 36 heures ou de 18 à 30 heures ou pendant une durée comprise dans toute plage définie par une combinaison de ces bornes.

La mise en contact est réalisée à une température de plus de 70°C et d'au plus égale à la température d'ébullition de la solution alcaline, par exemple à une température supérieure à 70°C et inférieure ou égale à 110°C, supérieure à 70°C et inférieure ou égale à 100°C, supérieure à 70°C et inférieure ou égale à 95°C ou dans toute plage définie par une combinaison de ces bornes, mais supérieure à 70°C. Ces gammes de température peuvent être combinées à chacune des durées précédemment définies. En règle générale, plus la température sera élevée, plus la durée sera courte.

La présence de dioxygène dissous peut résulter d'une mise en contact de la solution alcaline avec de l'air ou du dioxygène, ou encore d'un barbotage d'air ou de dioxygène dans la solution alcaline. Notamment, la mise en contact de la solution alcaline avec de l'air ou du dioxygène peut avantageusement être réalisée avec un renouvellement permanent de l'air ou du dioxygène en contact avec la solution, par exemple par un dispositif de recirculation.

Cette mise en contact ou ce barbotage sont de préférence réalisés avant la mise en contact de la solution alcaline avec les surfaces métalliques à traiter.

La mise en contact des surfaces métalliques est de préférence réalisée par remplissage des capacités métalliques formées de ces surfaces métalliques avec la solution préalablement préparée (i.e. contenant de l'oxygène dissous). La solution est ensuite injectée régulièrement ou en continu dans les capacités à traiter, de préférence avec une recirculation permanente. La teneur en O₂ dissous peut être contrôlée dans un bac de recirculation, et un barbotage d'air ou d'O₂ contrôlé en ce sens. Notamment, dans le cadre d'un protocole industriel, de l'air ou de l'O₂ pourrait par exemple être introduit via une canne d'injection dans un bac tampon ou de recirculation car d'une part une simple aération pourrait ne pas être suffisante et d'autre part une telle aération entrainerait une déperdition énergétique.

Une fois le traitement appliqué, les capacités sont vidangées, par exemple à l'aide d'une purge à l'azote. Des étapes de dégazage finales des capacités (à l'azote) peuvent ensuite effectuées pour s'assurer qu'aucune poche de dioxygène gazeux ne reste coincée dans les capacités.

L'étape (iii) peut être mise en oeuvre sous une pression de 0 à 10 barg (barg : bar gauge ou bar relatif, dont le zéro correspond à la pression atmosphérique). La mise en contact sous pression permet d'obtenir une température plus élevée et de gagner en temps et en efficacité.

### Procédé de traitement du 1,3-butadiène

Le procédé selon l'invention comprend :
(a) une étape de passivation des surfaces métalliques de l'unité de traitement par mise en oeuvre du procédé de passivation selon l'invention,
(b) suivie d'une étape traitement d'une coupe contenant du 1, 3-butadiène.

Cette étape de traitement est notamment réalisée à chaud, à savoir à une température de 20 à 120°C

Dans un mode de réalisation, l'étape (b) de traitement est une étape de séparation du 1,3-butadiène, notamment par distillation, optionnellement précédée d'une étape de balayage au diazote.

L'étape de balayage au diazote est par exemple suffisante pour éliminer toute présence d'air à l'intérieur de l'unité de séparation.

L'étape de séparation peut être réalisée dans une unité de traitement, ici de séparation, comprenant une section de distillation extractive suivie d'une section de rectification comprenant au moins une colonne de purification du butadiène, notamment lorsque l'unité de séparation fait partie d'une unité butadiène. Un dépropaniseur peut être placé soit en amont, soit en aval de la section de distillation extractive.

Dans un autre mode de réalisation, l'étape de traitement d'une coupe contenant du 1,3-butadiène est une étape de production d'une coupe C3 ou C4 d'une unité de vapocraquage, l'unité de traitement comprenant alors un dépropaniseur pour produire une coupe C3 ou un débutaniseur pour produire une coupe C4.

### Description des figures

La figure 1 représente l'évolution du potentiel d'abandon des échantillons de l'exemple 1.
La figure 2 représente les courbes de polarisation cathodiques des échantillons de l'exemple 1.
La figure 3 représente l'évolution du potentiel d'abandon des échantillons de l'exemple 2.
La figure 4 représente les courbes de polarisation anodiques des échantillons de l'exemple 2.

### Exemples

### Essais électrochimiques

Des échantillons d'acier de grade P 235 GH (norme EN 10028-2 : 2017) ayant subi différents procédés de passivation ont été testés électrochimiquement.

Tous les essais électrochimiques ont été réalisés dans une enceinte en verre chauffée au moyen d'une plaque chauffante. La solution utilisée possède un pH faiblement alcalin (pH de 7 à 10) afin de ne pas détériorer le revêtement à la surface, et permettre sa caractérisation. Avant les essais, la solution a été systématiquement désaérée à l'argon dans un réservoir séparé puis transférée dans la cellule de test sans contact avec l'air.

Tous les essais d'électrochimie ont été réalisés à 50°C en plaçant l'électrode verticalement dans la cellule et sans agitation (arrêt du bullage d'Argon). L'électrode de référence utilisée est au calomel saturé et la contre électrode en platine. La cellule d'essai est équipée d'un réfrigérant à boules permettant de condenser la vapeur d'eau résultant de la chauffe de la solution.

### Conditions de mesure de potentiel d'abandon

Pour suivre l'évolution du potentiel libre, les échantillons traités ont été directement introduits dans la cellule, sans aucune préparation de surface. Dès introduction de la solution dans la cellule, le potentiel a été suivi en continu jusqu'à stabilisation. Par défaut une même durée d'immersion des échantillons a été utilisée (environ 16h soit une nuit) avant les essais potentiodynamiques.

### Conditions d'essais potentiodynamiques

Les parties anodiques et cathodiques des courbes de polarisation ont été établies séparément sur deux ou trois échantillons différents. La partie anodique a été déterminée en balayant le potentiel de -50mV par rapport au potentiel d'abandon (Eab) jusqu'à +0,85 V/SCE. La partie cathodique a quant-à-elle été établie en balayant le potentiel de +50 mV/Eab jusqu'à -1,2V/SCE. Dans les deux cas, la vitesse de balayage était 10 mV/min.

### Exemple 1

Plusieurs échantillons d'acier de grade P 235 GH (norme EN 10028-2 :2017) ont été soumis aux traitements suivants, dont les paramètres figurent dans le tableau 1.

**Tableau 1**

| Paramètres | | Ref | T1 | T2 |
|---|---|---|---|---|
| Dégraissage | Aération | sous air | | |
| | Durée | 8H | | |
| | Température | 65°C | | |
| | Composition | 0,5%m NaOH + 0,01%m surfactant | | |
| Décapage | Aération | sous air | | |
| | Durée | 8H | | |
| | Température | 50°C | | |
| | Composition | 3%m acide citrique + 0,2%m inhibiteur de corrosion + pH^{∼}4 via NH3 | | |

| Re-oxydation volontaire | | non | non | oui |
|---|---|---|---|---|
| Passivation | Aération | - | sous air | sous air |
| | Durée | - | 24H | 24H |
| | Température | - | 90°C | 90°C |
| | Composition | - | 3%m NaNO₂ | 3%m NaNO₂ |

L'aération mentionnée dans les paramètres de la passivation correspond à l'aération de la solution, par une mise en contact avec l'air ambiant. Cette aération a été maintenue lors de l'immersion des échantillons métalliques dans la solution.

La réoxydation volontaire a consisté à immerger l'échantillon dans une solution de NaCl de concentration comprise entre 40 et 60g/L, à température ambiante.

L'immersion durant 7 jours a été complétée par un séchage à l'air libre pendant 7 jours.

Le surfactant peut être un sel de sodium d'alcool linéaire, de longueur C12-C14, sulfaté et ethoxylé (par exemple CAS N° 68891-38-3).

L'inhibiteur de corrosion peut être un mélange de polyoxyethylène et de thiourée.

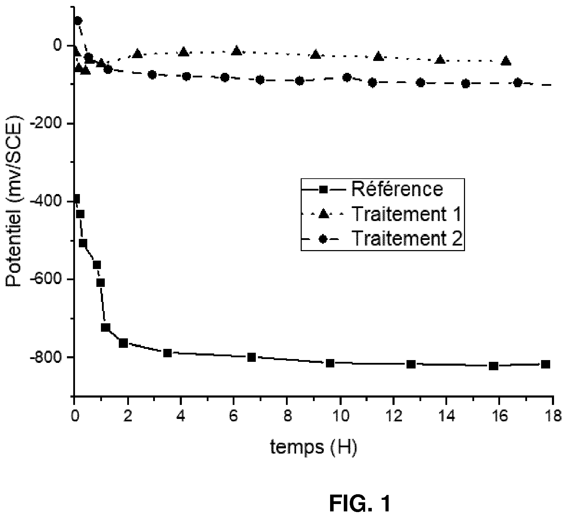

La figure 1 présente l'évolution des potentiels d'abandon : on observe un comportement très différent de l'acier non traité (Ref) comparativement aux pièces passivées. L'acier non traité est en effet actif avec un potentiel voisin de -800 mV/SCE tandis que les pièces traitées sont clairement passives avec des potentiels très élevés et supérieurs à -150 mV/SCE.

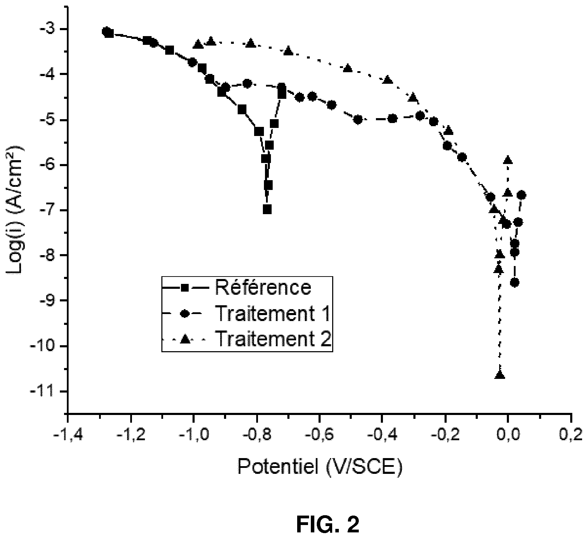

De manière analogue, la figure 2 présente les courbes de polarisation cathodiques typiquement obtenues sur les échantillons T1, T2 passivés comparativement à l'échantillon de référence non traité. On note une différence importante entre les deux types de traitement. Les pièces T1 présentent systématiquement un plateau qui n'est en revanche jamais observé sur les échantillons T2. On peut supposer que ce plateau caractérise la réaction de réduction directe de la couche de passivation qui serait donc d'une nature différente suivant qu'on réalise le traitement sur une surface préalablement décapée ou alors artificiellement oxydée. Le courant mesuré est également un ordre de grandeur plus faible sur le T1 par rapport au T2.

### Exemple 2

Plusieurs échantillons d'acier de grade P 235 GH (norme EN 10028-2 :2017) ont été soumis aux essais de passivation suivants.

Deux types de traitement T1A et T1B ont été réalisés, dont les paramètres sont rassemblés dans le tableau 2.

L'aération mentionnée dans les paramètres de la passivation correspond à l'aération de la solution, par une mise en contact avec l'air ambiant. Cette aération a été maintenue lors de l'immersion des échantillons métalliques dans la solution.

Le surfactant et l'inhibiteur de corrosion sont les mêmes que ceux de l'exemple 1.

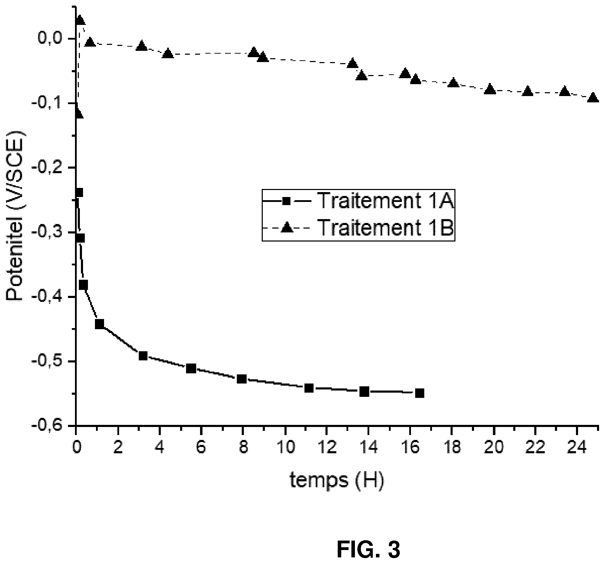

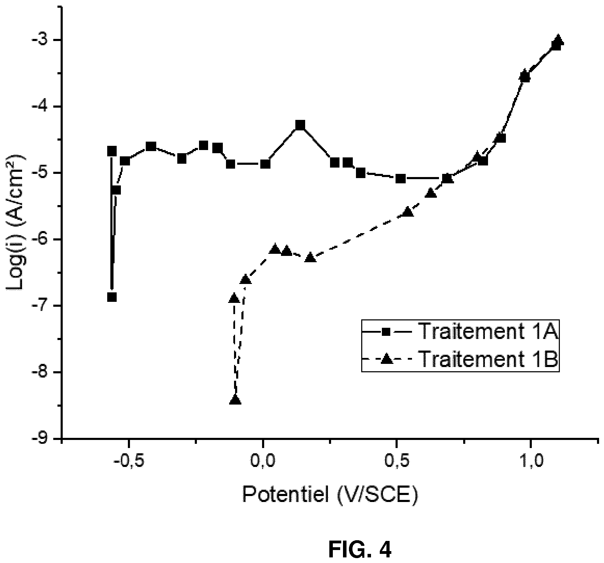

Les Figures 3 et 4 présentent les résultats obtenus.

**Tableau 2**

| Paramètres | | T1A | T1B |
|---|---|---|---|
| Dégraissage | Aération | sous air | |
| | Durée | 8H | |
| | Température | 65°C | |
| | Composition | 0,5%m NaOH + 0,01%m surfactant | |
| Décapage | Aération | sous air | |
| | Durée | 8H | |
| | Température | 50°C | |
| | Composition | 3%m acide citrique + 0,2%m inhibiteur de corrosion + pH^{∼}4 via NH₃ | |

| Re-oxydation volontaire | | non | non |
|---|---|---|---|
| Passivation | Aération | Sous diazote | sous air |
| | Durée | 24H | 24H |
| | Température | 90°C | 90°C |
| | Composition | 3%m NaNO₂ | 3%m NaNO₂ |

Lorsque le traitement est réalisé « en milieu désaéré » (traitement de référence T1A), autrement dit avec une solution de passivation sans dioxygène dissous (ou avec une teneur en dioxygène inférieure à 200ppb en masse), le potentiel d'abandon chute rapidement au début de l'immersion et se stabilise autour de -550 mV/SCE (fig.3). La courbe de polarisation anodique obtenue (fig. 4) est caractéristique d'un échantillon actif (pic d'activité présent au voisinage du potentiel d'abandon et courant de pic supérieur à 10 µA/cM²).

Le traitement réalisé « en milieu aéré » (T1B), autrement dit avec une solution de passivation comportant du dioxygène dissous en une teneur d'au moins 200ppb masse, conduit à un potentiel d'abandon supérieur à -100 mV/ECS (fig.3). La courbe de polarisation anodique est caractéristique d'un échantillon passif (potentiel de corrosion élevé, plateau de passivité, courant de passivité de l'ordre du µA/cm²) (fig. 4).

Ces résultats montrent l'importance de réaliser le traitement de passivation « en milieu aéré ». La courbe de polarisation anodique ne présente plus le pic d'oxydation vers -340mV/ECS. Ce pic correspondrait à l'oxydation d'un oxyde de fer formé lors du traitement de surface et qui nécessiterait donc la présence d'oxygène pendant le traitement.

## Revendications

1. Procédé de passivation des surfaces métalliques d'une unité de traitement d'une coupe contenant du 1,3-butadiène, comprenant les étapes suivantes :
(i) une étape de décapage dans laquelle lesdites surfaces métalliques sont mises en contact avec une solution acide présentant un pH de 3 à 5 constituée d'une solution aqueuse d'acide citrique et d'ammoniaque, et optionnellement d'un ou plusieurs inhibiteurs de corrosion, à une température comprise dans la plage allant de 50°C à 70°C, pendant une durée suffisante pour éliminer les oxydes de fer présents sur lesdites surfaces métalliques,
(ii) une étape d'évacuation de la solution acide ou de neutralisation de la solution acide par une base, de préférence de l'ammoniaque,
(iii) une étape de passivation dans laquelle lesdites surfaces métalliques préalablement traitées par la solution acide sont mises en contact avec une solution alcaline constituée d'une solution aqueuse de nitrite de sodium contenant du dioxygène dissous, pendant 12 à 48 heures à une température supérieure à 70°C, la solution aqueuse de nitrite de sodium contenant de 0,3 à 10% en masse de nitrite de sodium et de 200 ppb en masse de dioxygène à la limite de solubilité du dioxygène dans la solution alcaline.

2. Procédé de passivation selon la revendication 1, dans lequel l'étape (iii) est mise en oeuvre par mise en contact des surfaces métalliques avec la solution alcaline en présence d'air ou de dioxygène ou par barbotage d'air ou de dioxygène dans la solution alcaline.

3. Procédé de passivation selon la revendication 1 ou 2, dans lequel l'étape (iii) est mise en oeuvre à une température supérieure à 70°C et inférieure ou égale à la température d'ébullition de la solution alcaline.

4. Procédé de passivation selon l'une quelconque des revendications 1 à 3, dans lequel l'étape (iii) est mise en oeuvre à une pression de 0 à 10 barg.

5. Procédé de passivation selon l'une quelconque des revendications 1 à 4, dans lequel au cours de l'étape (i) lesdites surfaces métalliques sont mises en contact avec la solution acide pendant 6 à 12 heures.

6. Procédé de passivation selon l'une quelconque des revendications 1 à 5, dans lequel les surfaces métalliques sont soumises à une étape de dégraissage préalablement à l'étape (i) de décapage.

7. Procédé de traitement d'une coupe contenant du 1,3-butadiène dans une unité de traitement, comprenant :
(a) une étape de passivation des surfaces métalliques de l'unité de traitement par mise en oeuvre du procédé de passivation selon l'une quelconque des revendications 1 à 6,
(b) suivie d'une étape de traitement d'une coupe contenant du 1, 3-butadiène.

8. Procédé selon la revendication 7, dans lequel l'étape (b) de traitement est une étape de séparation du 1,3-butadiène, optionnellement précédée d'une étape de balayage au diazote.

9. Procédé selon la revendication 7, dans lequel l'étape (b) de traitement est une étape de production d'une coupe C3 ou C4 d'une unité de vapocraquage, l'unité de traitement comprenant un dépropaniseur pour produire une coupe C3 ou un débutaniseur pour produire une coupe C4.
